# EUROPEAN PATENT APPLICATION

(11) **EP 2 873 444 A2**
(43) Date of publication of application: **20.05.2015**
(21) Application number: 14192613.9
(22) Date of filing: 11.11.2014
(51) Int. Cl.: A63B 24/00, G06F 19/00

(54) **Virtual reality based rehabilitation apparatuses and methods**

(30) Priority: 13.11.2013 US 201314078611
(71) Applicant: Motorika Ltd., Hamilton HM 12 (BM)
(72) Inventor: Zohar, Ester, 5322902 Givataim (IL); Binyamini, Gad, 4587000 Moshav Hagor (IL); Zohar, Ron, 5310102 Givataim (IL); Shemer, Moran, 5310102 Givataim (IL)
(74) Representative: Dennemeyer & Associates S.A.

(57) **Abstract**

A method of measuring a user's range of motion, comprising: tracking user movement of at least one body portion using at least one sensor; correlating the tracked user movement to an avatar of the user in a virtual environment using at least one controller; displaying on an output device the summation of tracked user movement graphically in the virtual environment such that user movement with respect to the user is represented in the virtual environment as avatar movement with respect to the avatar, in the same relative relationship.

## Description

### FIELD AND BACKGROUND OF THE INVENTION

The present invention, in some embodiments thereof, relates to rehabilitation and/or exercise and, more particularly, but not exclusively, to a virtual reality-based system for rehabilitation and/or exercise and/or methods of implementation and/or use.

There are ever-increasing "natural" human interfaces for multimedia and computer systems. These interfaces use a dedicated sensor (e.g. Nintendo® Wii remote, Microsoft® Kinect) which captures three dimensional ("3D") positions of the hand and the body of a person and transforms them to 3D coordinates in some reference system.: Typically, the user is required to interact with a two-dimensional display, for example a television. Typical interaction involves pressing virtual buttons or selecting virtual objects on the screen. However, due to perceptual challenges, it is difficult to convert the 3D coordinates of the user's hand to a position within the image displayed on the screen in a way that is truly natural to the user. Thus, currently known interfaces (for example: those offered by Microsoft® in conjunction with PrimeSense™) require a two-step process to click an on-screen button. Initially, an icon of a hand (or another two-dimensional mark) is displayed somewhere on the screen. Movement of the user's hand in the real world to the right causes the hand icon to move to the right on the screen, and similarly in the other directions. When the virtual hand is on top of an intended button on the screen, the button changes its appearance to express to the user that the hand is indeed on top of it, only then the user is expected to perform another gesture to actually "press" the button.

### SUMMARY OF THE INVENTION

According to aspect of some embodiments of the invention, there is provided a method for measuring a user's range of motion. In an embodiment of the invention, at least one sensor is used to measure user motion in at least one dimension. Optionally, movement measurement is in two or three or more dimensions. In an embodiment of the invention, tracked user movement is correlated to an avatar in a virtual environment. In an embodiment of the invention, user movement with respect to the user is represented in the virtual environment as avatar movement with respect to the avatar, in the same relative relationship.

The user movement of the avatar in the virtual environment is displayed graphically in the virtual environment, in an embodiment of the invention. Optionally, a summation or aggregate of the tracked user motion is displayed, wherein it is graphically displayed to the user anywhere the avatar has moved in the virtual environment during tracking. Optionally, only some of the tracked user motion is displayed.

According to an aspect of some embodiments of the invention there is provided a method for the direct selection of an object in a virtual reality environment. In an embodiment of the invention, the method includes a measurement phase to determine a patient's range of motion. In an embodiment of the invention, the method includes an object display phase, wherein objects are placed in the virtual reality environment at least partly based on the patient's measured range of motion.

In an embodiment of the invention, the method is suitable for use in computerized systems for physical therapy since it requires the patient to stretch to the limits of the patient's range of motion.

According to an aspect of some embodiments of the invention there is provided a virtual reality based rehabilitation system which adapts games and/or exercises based on the measured range of motion of the patient. In an embodiment of the invention, objects within the games and/or exercises are displayed to the user near the outer limits of the patient's measured range of motion in order to encourage the patient to push the limits of the patient's motor and/or cognitive abilities.

In some embodiments of the invention, games are modified on the fly based on range of motion measurements made during the patient's exercise.

In some embodiments of the invention, patient performance and/or progress is assessed and/or reports are generated based on the performance and/or progress assessment.

According to an aspect of some embodiments of the invention there is provided a virtual reality based rehabilitation system which stimulates an exercising patient in order to keep the patient mentally engaged during exercise.

According to an aspect of some embodiments of the invention, there is provided a method for calibrating depth as perceived by a user in a virtual reality environment. In an embodiment of the invention, the user's range of motion is measured in the virtual reality environment. Optionally, at least one sensor is used to measure user motion. In an embodiment of the invention, user motion is measured in one, two, three or more dimensions. In an embodiment of the invention, the measured range of motion is saved, optionally to be processed by a controller. In an embodiment of the invention, the user's measured range of motion is used to determine where in the virtual reality environment objects are displayed to the user in the environment. Optionally, the environment itself is altered (e.g. background images are closer or farther away with respect to the user or the user's avatar) based on the range of motion measurements. In an embodiment of the invention, the virtual reality environment is customized for each user, based on their measured range of motion, even if the game and/or environment is generally the same.

There is provided in an exemplary embodiment of the invention, a method of measuring a user's range of motion, comprising: tracking user movement of at least one body portion using at least one sensor; correlating the tracked user movement to an avatar of the user in a virtual environment using at least one controller; displaying on an output device the summation of tracked user movement graphically in the virtual environment such that user movement with respect to the user is represented in the virtual environment as avatar movement with respect to the avatar, in the same relative relationship.

In an exemplary embodiment of the invention, the method further comprises storing the tracked user movement in a database.

In an exemplary embodiment of the invention, the same type of coordinate system is used for the user and the avatar. Optionally, the coordinate system type is a spherical coordinate system. Optionally, the same relative relationship is defined by the coordinate system.

In an exemplary embodiment of the invention, avatar is displayed on the output device from behind, in the third person.

In an exemplary embodiment of the invention, graphically displayed summation of tracked user movement is displayed relative to a reference point. Optionally, the reference point is located in a body plane of the avatar. Optionally, the reference point is at or near the center of the graphically displayed summation of tracked user movement. Optionally, the reference point is determined on the fly.

In an exemplary embodiment of the invention, the graphic display of the summation of tracked user movement is a bubble where the bubble surface represents the limit of user movement.

In an exemplary embodiment of the invention, the method further comprises encouraging the user to expand the graphically displayed summation of tracked user movement.

In an exemplary embodiment of the invention, the displayed summation of tracked user movement is related to the currently estimated range of motion of the user.

There is further provided in an exemplary embodiment of the invention, a virtual reality based rehabilitation system, comprising: at least one sensor for detecting and measuring the motion of a patient; at least one software programmed controller which adapts a displayed virtual reality environment based on the measured motion of the patient; and, at least one output device.

In an exemplary embodiment of the invention, the system further comprises a database for storage of at least one of measured motion of at least one patient, a virtual reality environment, a billing code, an exercise, a game, patient information, a report, and software.

In an exemplary embodiment of the invention, the system further comprises at least one user interactive device.

In an exemplary embodiment of the invention, at least one user interactive device is at least one of an elbow support and a ball.

In an exemplary embodiment of the invention, at least one user interactive device is a glove.

In an exemplary embodiment of the invention, the system further comprises a connection to at least one external communications network. Optionally, at least one component is remotely located from the patient.

In an exemplary embodiment of the invention, the controller processes measured patient range of motion to display objects in a virtual reality environment to the patient within the range of motion, near the limit of range of motion or outside the range of motion.

In an exemplary embodiment of the invention, the controller presents a game which challenges the patient's cognitive and movement abilities in combination.

In an exemplary embodiment of the invention, the controller assesses patient progress based on saved sensor data of the measured motion of the patient.

There is further provided in an exemplary embodiment of the invention, a method for keeping a patient engaged during virtual reality based rehabilitation, comprising: selecting a game for the patient in accordance with a rehabilitation plan; exercising the patient; and, presenting objects to the patient in a virtual reality environment during exercising which require patient interaction.

In an exemplary embodiment of the invention, the method further comprises measuring the patient's range of motion at least one of before, during and after exercising.

In an exemplary embodiment of the invention, presenting objects to the patient is at least partly based on the measuring.

In an exemplary embodiment of the invention, the method further comprises tracking patient engagement with the game and adapting the presenting to the patient based on the tracking in order to enhance patient engagement.

In an exemplary embodiment of the invention, the method further comprises involving the upper body of the patient in the exercising to enhance patient engagement.

There is further provided in an exemplary embodiment of the invention, a method for calibrating depth as perceived by a user in a virtual reality environment, comprising: measuring a user's range of motion using at least one sensor; saving the measured range of motion in a database; and, adapting the depth of a virtual reality environment at least partly based on the measured range of motion using a software programmed controller.

In an exemplary embodiment of the invention, the adapting varies depending on the user.

There is further provided in an exemplary embodiment of the invention, a method for direct selection of an object in a virtual reality environment, comprising: measuring a patient's range of motion using at least one sensor; and, displaying the object in the virtual reality environment at least partly based on the measured range of motion processed by a software programmed controller, wherein the displayed object is directly selected by an avatar which represents the patient in the virtual reality environment and wherein the avatar movement is controlled by the patient.

In an exemplary embodiment of the invention, displaying occurs at least partly based on a rehabilitation or exercise plan for the patient.

In an exemplary embodiment of the invention, the object is displayed at the outer limits of the patient's range of motion to encourage the patient to push the patient's abilities.

In an exemplary embodiment of the invention, measuring and displaying are performed on the fly, as the patient is moving.

In an exemplary embodiment of the invention, the avatar is displayed from behind, in the third person.

In an exemplary embodiment of the invention, patient movement with respect to the patient is represented in the virtual environment as avatar movement with respect to the avatar, in the same relative relationship. Optionally, patient movement with respect to the patient and avatar movement with respect to the avatar are measured and correlated using the same type of coordinate system.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

Implementation of the method and/or system of embodiments of the invention can involve performing or completing selected tasks manually, automatically, or a combination thereof. Moreover, according to actual instrumentation and equipment of embodiments of the method and/or system of the invention, several selected tasks could be implemented by hardware, by software or by firmware or by a combination thereof using an operating system.

For example, hardware (e.g. "controller") for performing selected tasks according to embodiments of the invention could be implemented as a chip or a circuit. As software, selected tasks according to embodiments of the invention could be implemented as a plurality of software instructions being executed by a computer using any suitable operating system. In an exemplary embodiment of the invention, one or more tasks according to exemplary embodiments of method and/or system as described herein are performed by a data processor or controller, such as a computing platform for executing a plurality of instructions. Optionally, the data processor includes a volatile memory for storing instructions and/or data and/or a non-volatile storage, for example, a magnetic hard-disk and/or removable media, for storing instructions and/or data. Optionally, a network connection is provided as well. A display and/or a user input device such as a keyboard or mouse are optionally provided as well.

### BRIEF DESCRIPTION OF THE DRAWINGS

The patent or application file contains at least one drawing executed in color. Copies of this patent or patent application publication with color drawing(s) will be provided by the Office upon request and payment of the necessary fee.

Some embodiments of the invention are herein described, by way of example only, with reference to the accompanying drawings. With specific reference now to the drawings in detail, it is stressed that the particulars shown are by way of example, are not necessarily to scale, and are for purposes of illustrative discussion of embodiments of the invention. In this regard, the description taken with the drawings makes apparent to those skilled in the art how embodiments of the invention may be practiced.

In the drawings:
FIG. 1 is a block diagram of a system for implementing a virtual reality direct selection method, in accordance with an exemplary embodiment of the invention;
FIG. 2 is a flowchart of a virtual reality direct selection method, in accordance with an exemplary embodiment of the invention;
FIG. 3 is a flowchart of a method for measuring a user's range of motion, in accordance with an exemplary embodiment of the invention;
FIGs. 4A-4F are illustrations representing a user's virtual reality avatar defining a user's range of motion, in accordance with an exemplary embodiment of the invention;
FIG. 5 is a range of motion graph, in accordance with an exemplary embodiment of the invention;
FIG. 6 is a flowchart of a method for displaying objects in a virtual reality environment for direct selection, in an exemplary embodiment of the invention;
FIG. 7 is a block diagram of a rehabilitation system, in accordance with an exemplary embodiment of the invention;
FIG. 8 is a flowchart of a method of rehabilitation, in accordance with an exemplary embodiment of the invention;
FIG. 9 is a block diagram of a user motivation enhanced rehabilitation system, in accordance with an exemplary embodiment of the invention;
FIG. 10 is a flowchart of a method of providing user motivation enhanced rehabilitation, in accordance with an exemplary embodiment of the invention; and
FIGs. 11A-11E are screen shots of representative games, in accordance with exemplary embodiments of the invention.

### DESCRIPTION OF EXEMPLARY EMBODIMENTS OF THE INVENTION

The present invention, in some embodiments thereof, relates to rehabilitation and/or exercise and, more particularly, but not exclusively, to a virtual reality-based system for rehabilitation and/or exercise and/or methods of implementation and/or use.

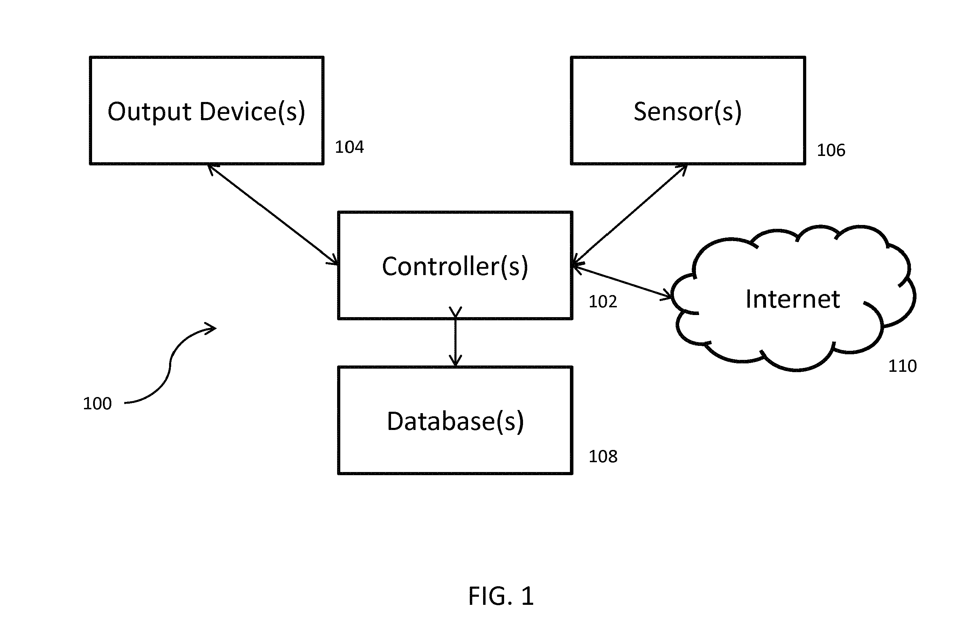

In an embodiment of the invention, a method or methods and related apparatuses are described, in relation to FIGS. 1-6, which facilitate interaction with a virtual reality environment and/or direct selection of objects and/or items in a virtual reality environment.

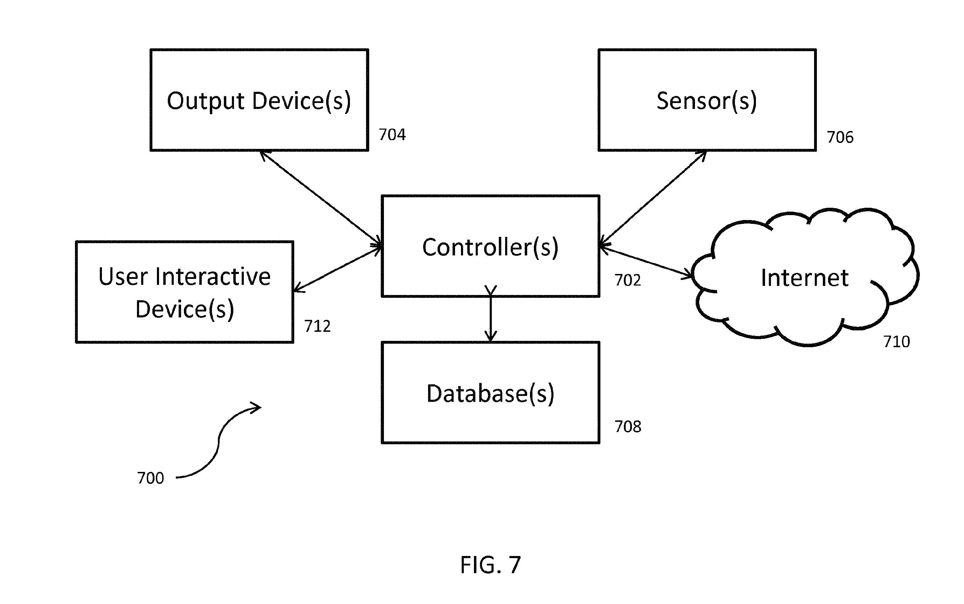

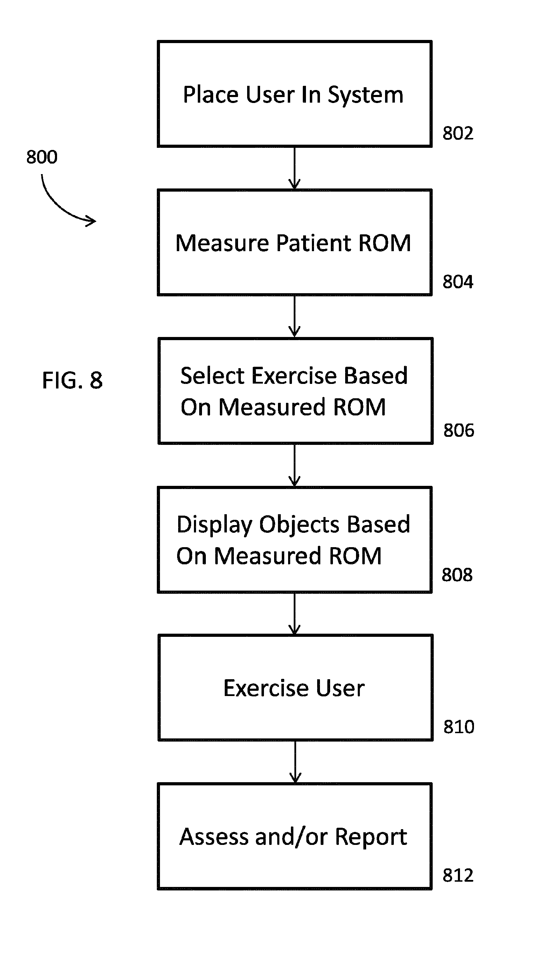

In some embodiments of the invention, these methods and apparatuses are employed as a component of a rehabilitation system, described in relation to FIGS. 7 and 8.

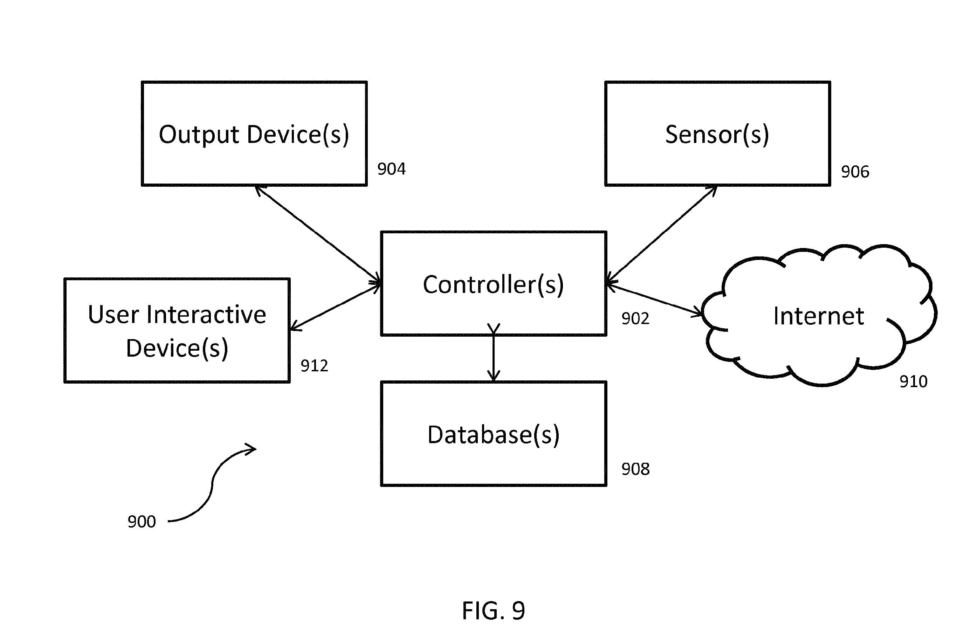

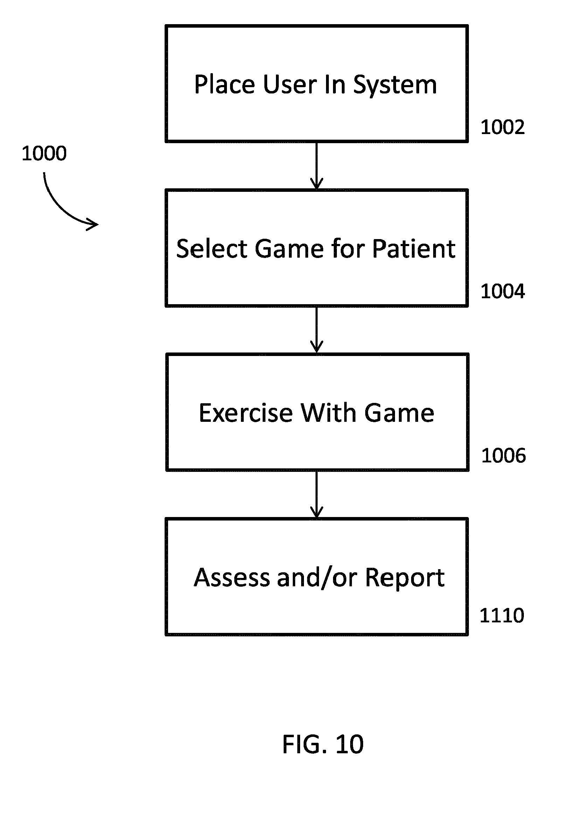

In some embodiments of the invention, these methods and apparatuses are employed as a component of a rehabilitation system which is configured to enhance user motivation and/or focus while the user is performing rehabilitation exercise. Such a system is described in relation to FIGS. 9 and 10.

Before explaining at least one embodiment of the invention in detail, it is to be understood that the invention is not necessarily limited in its application to the details of construction and the arrangement of the components and/or methods set forth in the following description and/or illustrated in the drawings and/or examples. The invention is capable of other embodiments or of being practiced or carried out in various ways.

FIG. 1 is a block diagram of a system 100 for implementing a virtual reality direct selection method, in accordance with an exemplary embodiment of the invention. In an embodiment of the invention, a controller 102 is provided to system 100 in operative communication with at least one output device 104, at least one sensor 106, and/or at least one database 108. In some embodiments of the invention, controller 102 is a processer or software programmed computer. In some embodiments of the invention, at least one sensor 102 senses movement in one, two, three or more dimensions, exemplars including a Nintendo® Wii remote or a Microsoft® Kinect camera. Optionally, more than one sensor 106 is used. Optionally, a plurality of sensors 106 are used, and optionally at more than one angle of incidence to the patient. In some embodiments of the invention, at least one output device 104 is, for example a video display, television or computer monitor.

In an embodiment of the invention, at least one component of the system 100 is operatively connected to an external communications network, for example the Internet. It should be understood that while the controller 102 is shown to be connected to the Internet, none, any one, some or all of the components could be connected to an external communications network 110. For example, at least one output device 104 and/or at least one sensor 106 could be remotely located via the Internet 110 from the controller 102. As another example, the at least one database 108 could be remotely located from the controller 102. As yet another example, it is conceived that the attending medical professional is remotely located.

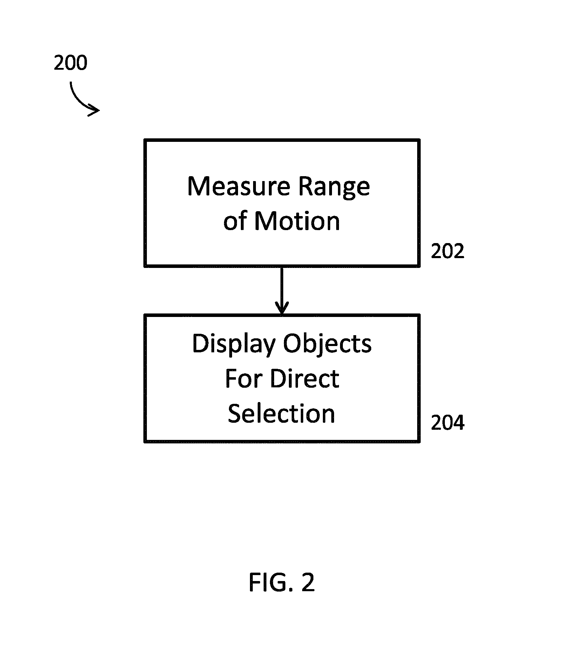

FIG. 2 is a flowchart 200 of a virtual reality direct selection method, in accordance with an exemplary embodiment of the invention. In an embodiment of the invention, the method comprises at least two actions, measurement of the patient's (or user's) range of motion (202) and displaying objects for direct selection by the patient (204).

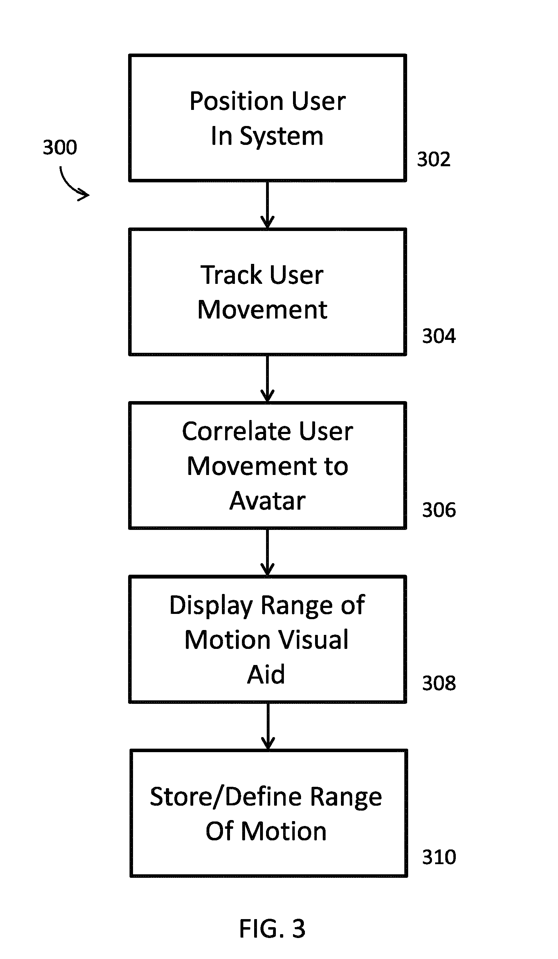

In an embodiment of the invention, measurement of range of motion (202) is used to interactively measure the range of motion in the virtual reality space of at least a portion (e.g. arm, hand, leg, finger) of the patient in a plurality of dimensions around the patient's body. Optionally, motion is measured (202) in three dimensions. Measurement (202) is shown and described in more detail with respect to FIGS. 3-5, *inter alia.*

Alternatively, additionally and/or optionally, other measurements of patient motor and/or cognitive ability are measured (202). For example, initiation time (how long it takes the patient to start moving), smoothness, efficiency of movement path, accuracy of movement, % compensation, number of repetitions, time exercised and/or time to complete the assigned task, are all characteristics which could be measured.

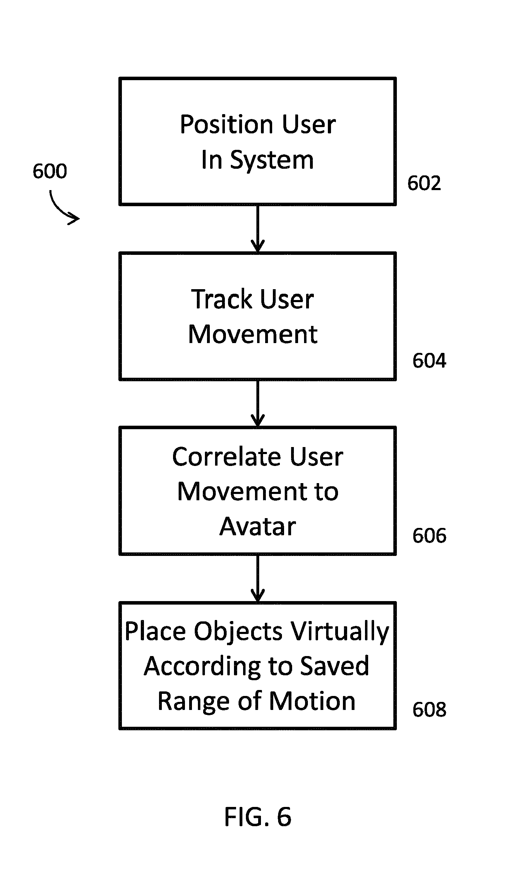

In an embodiment of the invention, objects are graphically displayed (204) in the virtual reality space at least partly based on the measured (202) patient range of motion. For example, in a rehabilitation setting where an objective is to increase the patient's range of motion, objects are displayed to the patient in the virtual reality setting near the limit of the patient's range, in order to force the patient to move to farther and farther distances away from the patient's body, thereby increasing range of motion in at least one dimension. Displaying (204) at least one object can also occur within the range of motion and/or outside the range of motion. Displaying (204), and the resultant effect on exercise, is shown and described in more detail with respect to FIG. 6, *inter alia.*

FIG. 3 is a flowchart 300 of a method for measuring a user's range of motion, in accordance with an exemplary embodiment of the invention. In an embodiment of the invention, measuring (202) range of motion of the user/patient is an interactive process in which patient movement of at least a portion of the patient's body is mapped in the virtual reality space with respect to an avatar of the patient, also present in the virtual reality space. It should be understood that in some embodiments of the invention, the relationship between movement of the patient and the patient's body in the "real" world corresponds to the relationship between movement in the virtual reality space and the avatar. Optionally, this correspondence is altered to achieve a result in the virtual space that is modified in relation to the movement result in real life. For some embodiments of the invention, each portion of the user is measured separately.

In an embodiment of the invention, the patient is positioned (302) in the system 100. Optionally, the patient is positioned (302) such that the vertical plane created by the patient's shoulders is approximately parallel to a display surface of the output device 104, and/or, optionally where the patient faces the display surface of the output device 104 and/or, optionally perpendicular to the direction of a remote sensor (described below). In an embodiment of the invention, the avatar is shown in the virtual environment with its back to the patient such that as the patient faces the display surface of the output device 104, movement by the patient in the real world corresponds directly to movement of the avatar in the virtual world. In such a scenario, the avatar is in essence a proxy for the patient in the virtual reality space and interaction in the virtual reality space should come naturally to the patient. Optionally, the avatar is placed in a different orientation with respect to the patient, for example as a mirror image. Optionally, the view presented on the output device 104 to the patient is in first person rather than third person. Optionally, the view perspective can be changed during measuring (202) and/or displaying (204).

In some embodiments of the invention, displaying (204) occurs right after measuring (202). Optionally, measuring (202) is performed, and then at a later time displaying (204) occurs using the saved measurement (202) data, for example during a subsequent rehabilitation and/or exercise session.

In an embodiment of the invention, patient movement in one, two or three dimensions is tracked (304) by the system 100, using for example at least one sensor 106. In an embodiment of the invention, the at least one sensor 106 is attached to the patient. Optionally, the at least one sensor 106 is not attached to the patient, but tracks patient movement remotely, for example the Microsoft® Kinect system. In some embodiments of the invention, the at least one sensor 106 is a combination of being attached to the patient and not, for example a remote sensor tracks motion of a part of the sensor that is attached to the patient, similar to the Nintendo® Wii system. In some embodiments of the invention, tracking (304) is continuous.

Tracked (304) patient movement is correlated (306) to the avatar located in the virtual reality space, in an embodiment of the invention. In an embodiment of the invention, a "skeleton" of the patient is acquired; typically this is a graph with nodes and edges, where nodes typically correspond to joints in the human body or other representative locations. The avatar is also assigned a "skeleton" such that nodes of the patient's actual skeleton are matched to corresponding nodes of the avatar's skeleton. In an embodiment of the invention, the process replicating movements of the patient as movements of the avatar is based on tracked (304) angles of movement between the nodes, and transferred from the patient's skeleton to the corresponding nodes of the avatar skeleton. In some embodiments of the invention, it suffices to transfer only angles since the lengths of the edges in the avatar skeleton are fixed and known. Note that these lengths are different from the lengths of the corresponding edges in the patient's skeleton (which represent the actual dimensions of the patient). In practice, when the patient straightens her arm, the avatar straightens its arm. When the patient raises her hand, the avatar figure raises its hand.

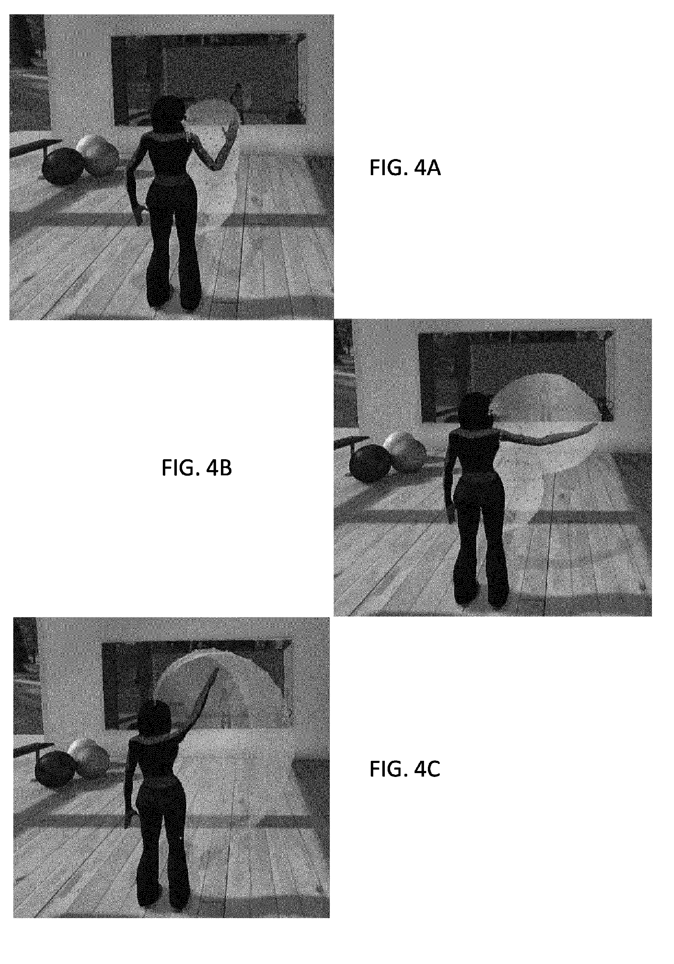

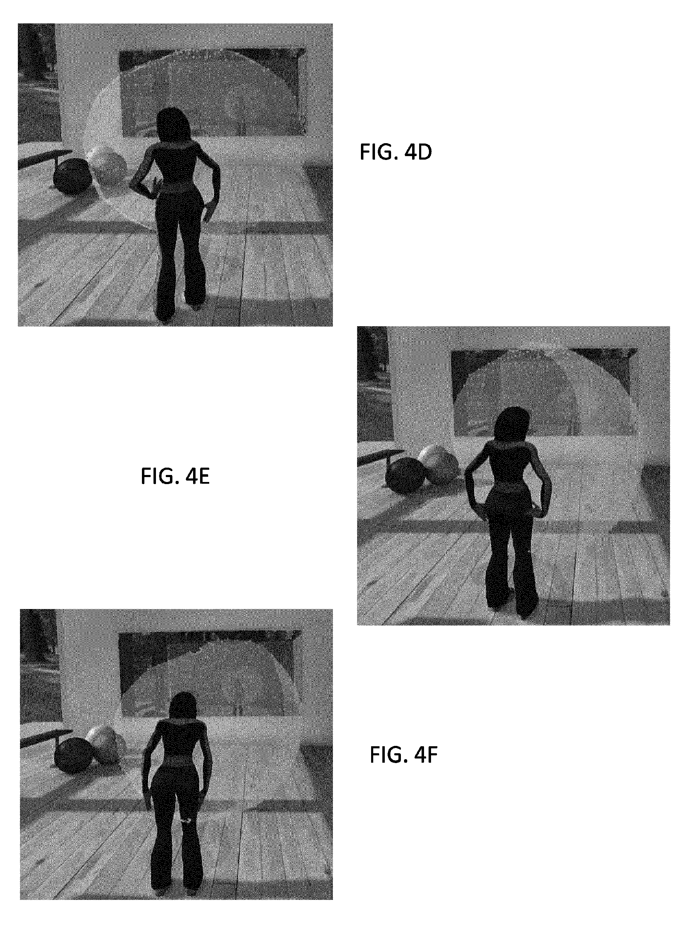

In an embodiment of the invention, as the patient moves, thereby causing the avatar to move in the virtual reality space, the tracked (304) patient's range of motion is displayed (308) visually in the virtual space. FIGS. 4A-4F are illustrations representing a user's virtual reality avatar defining a user's range of motion by displaying a (308) a visual aid or graphical display (*e.g.* a bubble) of a summation or aggregate of the user's tracked motion, in accordance with an exemplary embodiment of the invention. Optionally, only a portion of the tracked motion is displayed graphically. Optionally, none of the tracked motion is displayed graphically. In an embodiment of the invention, the range of motion is displayed as a regular or irregularly shaped bubble where the exterior surface of the bubble is that farthest reach of the patient. In an embodiment of the invention, the surface expands whenever the avatar figure touches it, such that it represents the farthest positions reached by the patient. The patient is instructed to extend the surface as much as she can during measuring (202) in order to determine the patient's full range of motion. By examining the bubble which is created through patient movement, the displayed (308) surface allows the patient to understand in real time which angles have not been visited yet with her hand and/or to what extent (range) and thus, the patient inspired to try and expand the exterior surface of the bubble in those areas.

The bubble location is calculated relative to some reference point associated with the avatar figure. In some embodiments of the invention, for example if an arm is being tested, the reference point at the shoulder node of the arm under test in the avatar's body plane (this plane can be calculated using a vector product of the position difference vectors from shoulders to pelvis). In some embodiments, it is assumed by the system 100 that the body plane is the plane perpendicular to remote sensor direction, because the patient is standing in front of the remote sensor. Using this approximation, it is not required to explicitly calculate the plane using a vector product.

By setting a reference point, any motion by the patient can then be mapped using a three dimensional coordinate system (*e.g.* a spherical (phi, theta) system is a type of three dimensional coordinate system), in this example with the shoulder node as the center. Using a spherical body coordinate system, which defines phi angle for left and right movement (phi=90 degrees when the arm is perpendicular to the plane of the body) and theta angle defines the motion of the arm in the up and down directions (theta=90 degrees when the arm is perpendicular to the plane of the body). In an embodiment of the invention, the exterior bubble surface displayed represents in this coordinate system, for each pair of angles (phi, theta), the maximum range achieved for the tested arm of the avatar figure. While an arm is used as an example, the portion of the body being measured (202) could be virtually anything, such as a leg, a hand, a finger, a head, etc. it should also be understood that the reference point for the coordinate system need not be an actual joint, and can be, for example, the average position of a plurality of joints or a translated position with respect to a node.

In an embodiment of the invention, measuring (202) occurs while the patient is exercising (*i.e.* dynamic mapping), in the midst of a game, such that objects which are presented to the patient in the game can change location based on the in-game measurements (202) of the patient's range of motion, as opposed to relying on a previous recorded patient range of motion.

Referring to FIGS. 4A-4C, it can be seen how the surface expands due to the straightening of the avatar's arm and the movement of the straightened arm in the virtual space. The reference point for the movement bubble is the shoulder node on the side of the arm being moved (although because perfectly symmetrical motion of the arm around the shoulder joint is anatomically impossible, the reference point will not actually be at the center of the bubble created). In some embodiments of the invention, the reference point is selected such that it is as close to the center of the bubble as reasonably possible to reliably represent the range of motion. For example, if the patient cannot move her elbow from her waist, measuring the range of motion of the hand with respect to the elbow will produce more reliable results. Optionally, both are measured simultaneously (*i.e.* the range of motion of the hand with respect to the elbow and of the hand with respect to the shoulder). In some embodiments of the invention, the reference point is determined on the fly, based on real-time measurements of patient motion. In an embodiment of the invention using this real-time approach, the reference point for the range of motion will actually be the center of the bubble. This can be an iterative process where initially the reference point is the shoulder, and then based on the first iteration the reference point is selected elsewhere. This can also be done in a bootstrapped manner.

In some embodiments of the invention, different colors are used to show the patient where additional movement and/or stretching of the surface is possible and/or desirable. For example, red areas are determined by the system 100 as areas which are not fully explored by the avatar's motion, but green areas are maximized, or are close to being maximized. In an embodiment of the invention, this color visualization makes it easier for the patient to identify angles and/or ranges which require an additional effort.

Referring to FIGS. 4D-4G, it can be seen how the surface is referenced to the plane of the avatar's body as described above. In FIG. 4D, the avatar figure is turning its body to the left. In FIG. 4E, the avatar is turning its body to the right and in FIG. 4G, the avatar is taking a bow. It can be observed that the bubble, corresponding to measured (202) range of motion, moves accordingly. This is important, in an embodiment of the invention, because the exterior surface of the bubble represents actual motion constraints of the patient in the real world.

Once the range of motion of the patient has been tracked (304), correlated (306) and displayed (308), the range is stored (310) on the at least one database 110. Optionally, the range of motion is not correlated (306) and/or displayed (308) simultaneous to the tracking (304), for example if no output device 104 is available at the location where the measuring (202) is taking place and/or if exercise may occur later.

In an embodiment of the invention, the mapped surface of the bubble is calculated for the specific avatar figure used in the virtual reality environment, and therefore the defined bubble may depend on the avatar's arm length. Consequently, a different surface could be mapped for a different avatar even for the same patient, in an embodiment of the invention (*i.e.* measured values are normalized to the patient). In some embodiments of the invention, the avatar dimensions are the same regardless of the avatar chosen to represent the patient in the virtual world (*i.e.* avatar dimensions are normalized).

Notwithstanding the above, in some embodiments of the invention, the range of motion that is tracked (304) and/or stored (310) is in relation to the patient's actual body in the real world, and a correlating coordinate system.

In an embodiment of the invention, a metric ("P-ROM") for defining a patient's range of motion is established such that for each pair of angles (phi, theta) in the spherical coordinate system, the P-ROM is the ratio between the shoulder to hand distance and the sum of distances, shoulder to elbow and elbow to hand. In an embodiment of the invention, the P-ROM is a scalar value in the range 0-1, such that 1 corresponds to a full range of motion (arm fully stretched) and 0 corresponds to a very limited range of motion, for a given pair (phi, theta). This metric provides very similar values when computed in the avatar's body coordinate system and when computed in the patient's body coordinate system. This interesting invariance property seems to hold even though there is no direct relationship between the coordinate systems and the patient's bone lengths are different from the avatar's skeleton bone lengths.

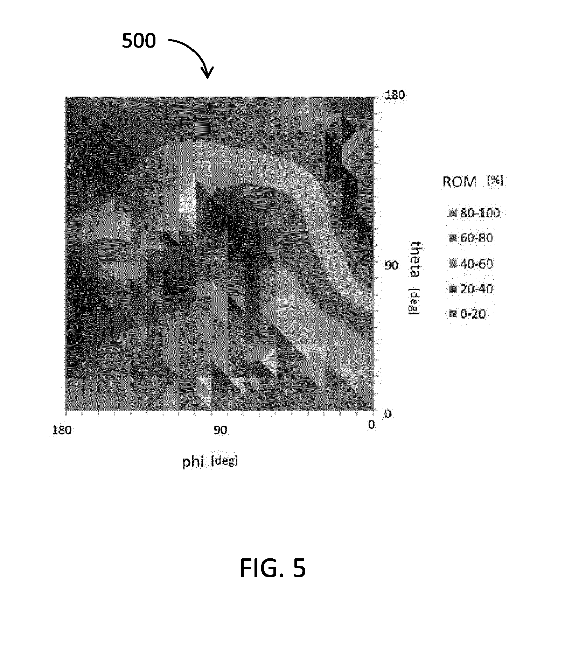

FIG. 5 is a range of motion graph 500 where 0 means that the arm is aimed to the right and 180 means that the arm is aimed to the left, in accordance with an exemplary embodiment of the invention. In this example, range of motion values are saved for the patient's right hand (in percentages). The X-axis represents the phi angle and the Y axis represents the theta angle, in the spherical coordinate system. It can be inferred from the details of the figure that the patient has a difficulty lifting a straight arm and has very limited range of motion beyond the shoulder level. Above this height (at angle theta = 90) a sharp decline is observed for the range of motion values for all phi angles.

FIG. 6 is a flowchart 600 of a method for displaying (204) objects in a virtual reality environment for direct selection, in an exemplary embodiment of the invention. In an embodiment of the invention, the patient is positioned (602) in the system 100. As with the measuring (202) phase, the patient is located so that the patient's shoulders are approximately parallel to a display surface of the output device 104 and/or are approximately perpendicular to the direction faced by a remote sensor, if there is one. In an embodiment of the invention, the avatar is displayed with its back facing the patient, in a third person fashion. Optionally, the view presented to the patient is in first person.

Similar to the measuring (202) phase, user movement is tracked (604) in the real world and correlated (606) to an avatar in the virtual environment, in an embodiment of the invention.

During patient use of the system 100, objects are placed (608) in the virtual reality environment in relation to the avatar as they would be placed in relation to the patient in the real world. That is, objects are placed at angles (phi, theta) in the avatar body coordinate system in a range, R, that is based on the real world values saved for the patient, in the patient body coordinate system, for these angles. It should be recalled that range of motion values, P-ROM, represent for each pair of (phi, theta) angles, the ratio between the shoulder to hand distance and the sum of distances, shoulder to elbow and elbow to hand (in the case of an arm being measured and/or exercised). In an embodiment of the invention, placing the object "in range R" based on range of motion requires, first, a computation of D (sum of distances, shoulder to elbow and elbow to hand) and then, placing the object in angles (phi, theta) in the avatar body coordinate system in range R where R= P-ROM(phi, theta) * D.

In this way, it is believed by the inventors that when an object is displayed at angles (phi, theta) in the avatar body coordinate system, the patient is able to seamlessly interpret these angles to her own body coordinate system and to move the avatar in the virtual environment as desired and/or as instructed using the objects as goals and/or motivation to achieve that movement.

As described elsewhere herein, particularly with respect to FIGS. 8 and 10, placing the objects near the edge of the patient's range of motion is highly desirable for exercise and/or rehabilitation. For example, while the patient is able to seamlessly interpret the angle of the object relative to the avatar, the range to the object may not be sufficiently observable. However, placing the object near the edge of feasible movement (or measured (202) movement) provides confidence to the patient as to the required range that should be met. This helps the patient to make a direct and decisive gesture in the (phi, theta) direction, which in turn brings the avatar's hand close to the object.

In an embodiment of the invention, representation of object range R in the avatar body coordinate system as described above is at least partially the result of the interesting invariance property described herein with respect to P-ROM values. In the absence of such an invariance property it would be difficult to establish an adequate range R for placing the object.

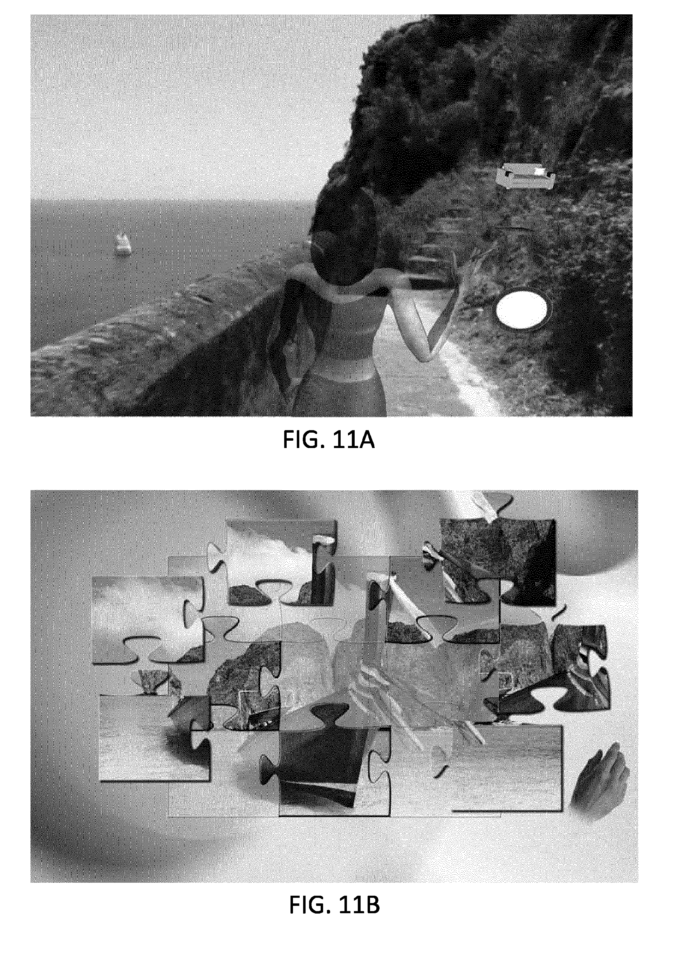

In some embodiments, where high accuracy is required for selection, it is recommended to provide the patient with additional indications to help her orient in space the range to the selectable object, such as drawing a line (shown in FIG. 11A) connecting the object of interest and the avatar hand, or any other interactive behavior that the selectable object may exhibit when the avatar's hand is approaching it.

FIG. 7 is a block diagram of a rehabilitation system 700, in accordance with an exemplary embodiment of the invention. Rehabilitation system 700 is a virtual reality based rehabilitation system which provides therapeutic activity and/or accurate measurement of post-stroke patients, in some embodiments of the invention. System is configured to provide an engaging, motivating environment where the motion of the avatar and/or limb displayed in the virtual world is a replication of the motion produced in the real world by the user/patient. In an embodiment of the invention, system 700 is designed to assess and/or improve movement of elderly, post-stroke and/or orthopedic impaired people. Optionally, system 700 is located at a physical therapy/occupational therapy clinic and/or in a long term care environment. In some embodiments of the invention, system 700 provides patients with a treatment program utilizing tailored therapy sessions and therapeutic games.

In an embodiment of the invention, the system 700 is provided with a software programmed controller 702, for example a computer. In an embodiment of the invention, controller 702 performs at least one of graphics processing, sensor monitoring, movement tracking, report generation, saving and/or calling for data, coordinating components of system 700, generating games and/or exercises to be played by the patient, determining where in the virtual environment objects should be placed based on patient P-ROM data, tracking patient movement in relation to the virtual environment and/or placed objects, etc.

At least one output device 704, for example a video display of some sort, is provided in operative communication with the controller 702 such that a virtual environment and/or exercises and/or games can be presented to the patient. Optionally, the output device 704 is a television. Optionally, the output device 704 is a computer screen. Optionally, the output device 704 is a portable device, like a mobile phone or deployable screen/projector system. In some embodiments of the invention, an output device 704 is a printer, for example to generate written reports about the patient and/or the system 700. In some embodiments of the invention, an output device 704, such as a speaker, supplies audio to the system 700 and/or to the patient using the system 700. For example, for exercises or games which synchronize movement to music and/or to provide sound effects for the games being played.

In some embodiments of the invention, at least one sensor 706 is provided to the system 700 for tracking patient movement. Movement of the patient is tracked in one, two or three dimensions, in some embodiments of the invention. In an embodiment of the invention, the at least one sensor 706 is placed proximal to and/or directed to sense in a direction perpendicular to the display surface of the output device. The at least one sensor 706 is optionally of the type described above with respect to FIG. 1. Optionally, more than one sensor 706 is used. Optionally, a plurality of sensors 706 are used, and optionally at more than one angle of incidence to the patient.

In some embodiments of the invention, at least one database 708 is provided to the system 700. Database could, for example, store games, exercises, patient data, reports, insurance billing codes, software, and/or other relevant information. Optionally, the database 708 is remotely located and communicates with the system 700 via a communications network, like the Internet 710.

In an embodiment of the invention, at least one user interactive device 712 is provided to the system 700. For example, an elbow support could be provided to support spastic/paralyzed patient limbs. Another example of a user interactive device 712 is a ball, which in some embodiments of the invention acts as an on/off switch and/or provides a clicking functionality for use in the virtual reality environment. In some embodiments of the invention, the ball is provided with sensing abilities, for example strength of user squeeze, acceleration, direction, location, orientation, etc.

As another example of at least one user interactive device 712, a glove is provided to measure the activity of a hand, for example whether the hand is open or closed and to what degree. Optionally, the glove provides movement sensing for at least one finger and/or a means for selecting objects (*e.g.* a clickable button).

In some embodiments of the invention, the user interactive device 712 is motorized. In some embodiments of the invention, the user interactive device 712 measures patient force, movement, acceleration, location, EMG of opposing muscles and/or other measurables and/or reports these to the controller 702 for analysis and/or processing.

In some embodiments of the invention, the user interactive device 712 is used to help measure patient spasticity during exercise.

In an embodiment of the invention, some or all of the components of the system 700 are mounted, for example on a stand. Optionally, the components of the system 700 are configured to be portable, with wheels or on stands with wheels for example. In some embodiments of the invention, an interface is provided for the user/patient and/or an attending medical professional to interact with the system 700. Optionally, the interface is the controller 702. Optionally, the interface includes a mouse and/or keyboard. In some embodiments of the invention, the "attending medical professional" is virtual and/or computer controlled.

In an embodiment of the invention, at least one component of the system 700 is operatively connected to a communications network 710, such as the Internet. In an embodiment of the invention, a connection to the network 710 provides access to enhanced game and/or exercise libraries. In some embodiments of the invention, a connection to the network 710 enables online games amongst multiple system 700 users, optionally in a plurality of locations. In some embodiments of the invention, a connection to the network 710 enables the system 700 to automatically and/or directly submit billing information, for example claims to insurance companies. In some embodiments of the invention, a connection to the network 710 allows for local user data/information to be compared to remote user data/information, for example for the construction of a comparative database and/or group/population statistics. In some embodiments of the invention, a connection to the network 710 enables remote software updating of the system 700. In some embodiments of the invention, a connection to the network 710 allows an "attending" medical professional to be remotely located from the patient. In some embodiments of the invention, a connection to the network 710 permits management access to reports and/or statistics across multiple rehabilitation locations. In some embodiments of the invention, a connection to the network 710 allows for online scheduling for use of the system 700.

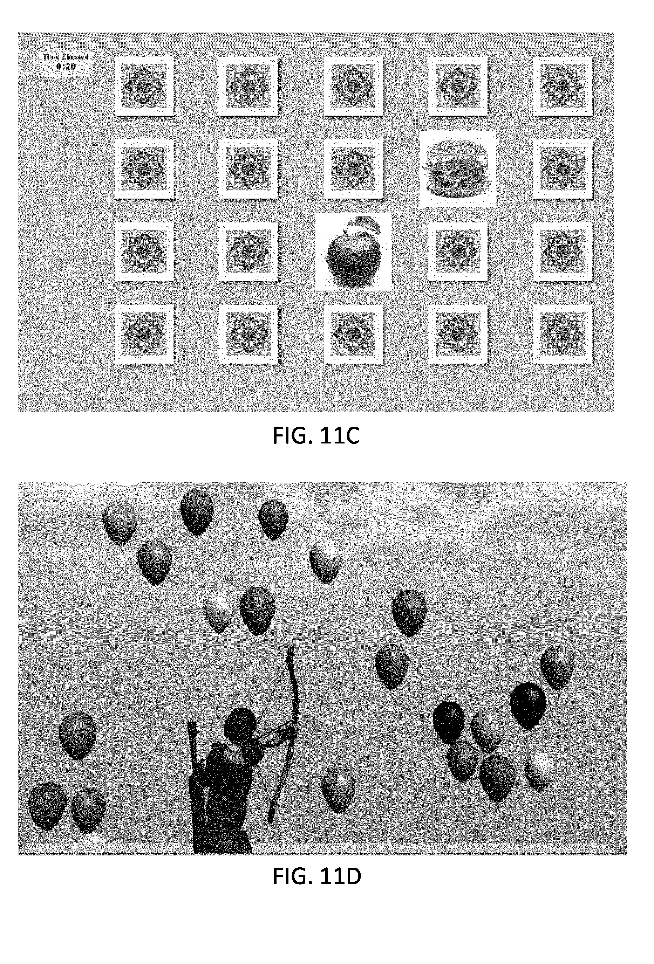

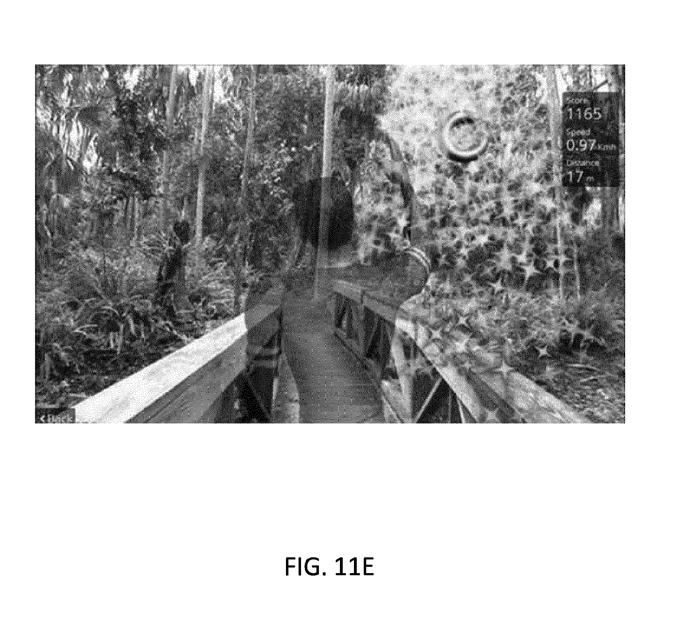

In an embodiment of the invention, the controller 702 is programmed to prompt the patient to improve patient motor function, for example after a stroke or some other orthopedic injury, and/or to provide some way to assess patient performance and/or progress. In some embodiments of the invention, the controller is programmed to offer games/exercises, such as described with respect to FIGS. 8 and 11, in which the patient participates and ideally causes improved movement and/or motor function in the patient.

In an embodiment of the invention, system 700 (or any of the other systems 100, 900 described herein) is located in a hospital, a clinic, nursing home or at the patient's home. In some embodiments of the invention, certain rehabilitation exercise is conducted in the more formal health care setting due to having more resources, for example leg rehabilitation may occur in hospitals and clinics, whereas hand rehabilitation could also at home and with less equipment. For example, in the home setting perhaps only the controller 702, the output device 704 and the sensor 706 are required and in a larger setting, such as a clinic, interactive user devices 712 like a treadmill or a robotic arm are added.

FIG. 8 is a flowchart 800 of a method of rehabilitation, in accordance with an exemplary embodiment of the invention. In an embodiment of the invention, a user/patient is placed (802) in the system 700. Although not required, the user faces the output device 704 such as described elsewhere herein. If the user is new to the system 700 (*i.e.* has not had a range of motion measured previously) then the patient's range of motion is measured (804) and recorded, for example as described with respect to FIGS. 2 and 3, and elsewhere herein. Optionally, the patient's range of motion is measured (804) in order to help determine if the patient has made progress over the previous session.

It is conceived, in an embodiment of the invention, that a wide range of virtual reality games and/or exercises are available to be presented by the system 700 which address or are capable of addressing different aspects of deficient patient range of motion. In an embodiment of the invention, exercises and/or games are selected (806) for presentation, optionally based on the measured (804) range of motion. As the exercise and/or game is presented to the patient, objects within the game are displayed (808) to the patient which are intended to force the patient to push the limits of their motor and/or mental abilities. The patient exercises (810) by progressing through the game or games which are selected (806) by the system 700 and/or an attending medical professional. Before, during and/or after exercise (810) patient performance is assessed (812) and/or at least one report is generated regarding the patient.

In an embodiment of the invention, various types of games can be selected (806) for the patient: assessment games, motion games, cognitive games, social games, activity of daily living games, games which cause the patient to repeat movements and/or games which force the patient to progress through a sequence of actions. Optionally, some games combine repetition and sequence. In some embodiments of the invention, assessment games measure a specific user ability. In some embodiments of the invention, motion games measure and/or exercise specific user movements. In some embodiments of the invention, cognitive games pair movement with a mental component. In some embodiments of the invention, activity of daily living games try to recreate real-life scenarios that would be encountered by the patient in day to day living, for example preparing a meal or brushing teeth.

Examples of games include putting dishes into a dishwasher or taking them out of the dishwasher (or in some cases, as many as they can in a given time frame), pouring liquids into cups and/or different kinds of containers, drinking from cups and/or different kinds of containers, putting or taking dishes out of a cabinet, hand washing dishes in a virtual sink, setting the table, cleaning a mirror, painting (e.g. walls), shaving, watering plants, folding clothes, putting clothes into a drawer or closet, rowing a boat and games involving balls (e.g. throwing, rolling).

Examples of games include preparing food (e.g. baking a cake, taking out/measuring ingredients, opening/closing containers, etc.), drinking, cutting pieces from an object (e.g. slicing a cake), eating, brushing teeth (including putting toothpaste on the toothbrush), washing a body part, brushing hair, turning on a light, turning off and/or setting an alarm clock, making a bed, opening and/or closing the curtains, opening and/or closing a window, picking up and/or dialing and/or hanging up a telephone, getting the avatar dressed, getting into a car, driving to a location, and walking the dog.

An example of a game is, in addition to some of the games already listed, shopping from a shopping list (pulling different items off the store shelves and/or putting them into the cart).

It should be understood that the above lists are examples only to give an idea of the types of games that could be offered, and that they are not exhaustive lists.

In an embodiment of the invention, exercises and/or games are selected to match to the particular body part and/or movement subset being rehabilitated. For example, in an embodiment of the invention where the objective is to rehabilitate the hand (and/or fine motor skills), a game is chosen which encourages the patient to pick up small objects in the virtual world and/or carefully place them down, optionally in small spaces or on small targets. Another exemplary game could involve drawing or tracing with one or more finger.

In some embodiments of the invention, game difficulty can be increased or decreased depending on the assessed need of the patient, for entertainment, to increase/decrease the cognitive load on the patient, to reduce or ease spasticity and/or to keep the patient mentally engaged (similar to what is described with respect to FIGS. 9 and 10) or for any other consideration.

Some games may be progressive, for example at lower levels only involving simple motor skills but at higher levels incorporating memory and/or cognitive skills in addition to motor skills. In some embodiments of the invention, games are set to music, for example if some actions should be performed in a certain timing or rhythm or if sound effects are to be provided to enhance engagement and/or enjoyment of the game.

In some embodiments of the invention, the games and/or exercises are modifiable or customizable so that the attending medical professional can manually adjust object locations, desired ranges of motion, and other exercise and game variables.

In an embodiment of the invention, desired exercise movements can be mapped to game control movements. Optionally, this is performed in the virtual reality environment.

In some embodiments of the invention, assessment and/or reporting (812) is performed before, during and/or after exercise (810). Optionally, assessment and/or reporting (812) occurs periodically, for example daily, weekly and/or monthly. In some embodiments of the invention, the games which are selected (806) for the patient are the same from session to session so that assessment (812) can be in comparison of the same exercises. In some embodiments of the invention, measurement (202) of range of motion is performed in order to assist with the assessment of patient progress and/or performance.

In an embodiment of the invention, reports are generated, optionally based on the assessment, to provide information on patient progress and/or performance. In some embodiments of the invention, the report includes a history of patient progress and/or performance, comparing a plurality of sessions. Reports optionally include measurements of patient motor and/or cognitive ability, for example range of motion, initiation time (how long it takes the patient to start moving), smoothness, efficiency of movement path, accuracy of movement, % compensation, number of repetitions, time exercised and/or time to complete the assigned task.

FIG. 9 is a block diagram of a user motivation enhanced rehabilitation system 900, in accordance with an exemplary embodiment of the invention. System 900 is designed, in an embodiment of the invention, to help improve the patient compliance during a rehabilitation session and/or to enhance the rehabilitation session by providing an interactive virtual reality space which augments the real world exercise setting. In an embodiment of the invention, it is conceived that the virtual reality component of the system 900 engages the patient, forestalling or preventing patient boredom, distraction and/or exhaustion (both physically and mentally) during exercise. In some embodiments of the invention, the system 900 is used to rehabilitate patients with neurology injury in addition to motor/orthopedic injury.

In a particular example, gait rehabilitation is often directed at exercising the lower body. It is often the case during gait rehabilitation that patients find ways to stay entertained, for example reading the newspaper, talking on the phone, and the like. None of these traditional methods of passing time during gait rehabilitation are particularly useful or contributory to gait rehabilitation, especially since the patient is not mentally engaged in the exercise session.

In some embodiments of the invention, the system 900 is configured to provide games in a virtual reality environment to the patient during rehabilitation which encourages the patient to remain engaged during the session. Not unlike the system 700 of FIG. 7, a software programmed controller 902 presents games to the patient via at least one output device 904 and, optionally, also through a user interactive device 912, such as a treadmill and/or hand grips. At least one database 908 is provided to the system 900 for storage of games, patient information, billing codes, etc. In an embodiment of the invention, at least one sensor 906 is used to measure the patient's range of motion, such as described elsewhere herein, and/or to track patient movements as the patient participates in the game. Optionally, the system 900 is connected to an external communications network 910, such as the Internet. It should be understood that some or all of the described components of system 900 are similar to those already described with respect to systems 100 and 700, and in the interests of brevity are not re-described here.

In some embodiments of the invention, some or all of the components (902, 904, 906, 908, 912) are configured to be portable and/or come as a kit, which can be retrofitted onto already existing rehabilitation machines or systems.

FIG. 10 is a flowchart 1000 of a method of providing user motivation enhanced rehabilitation using system 900, in accordance with an exemplary embodiment of the invention. In an embodiment of the invention, a patient is placed (1002) into the system 900 in an appropriate position to provide exercise to the patient. For example, in the case of a treadmill the patient is placed on the treadmill. Optionally, the patient is provided support as needed for deficient body parts, for example the patient could be at least partially suspended and/or one or both arms could be supported (this also applies to the other systems and/or methods described herein). In an embodiment of the invention, the patient is also placed (1002) in a position whereby the patient can interact with at least one output device 904 and/or can interact with at least one interactive user device 912.

In an embodiment of the invention, a game is selected (1004) for the patient which correlates to the rehabilitation program which is being provided to the patient. For example, in a gait training scenario, the game selected (1004) may involve walking along a path and/or climbing steps. Optionally, at least one of the output device 904 and/or user interactive device 912 are coordinated with the game during exercise (1006) such that the speed of walking, for example, is timed to the advancement down the path in the virtual world. Optionally, there is tactile feedback for actions performed in the game.

In some embodiments of the invention, during exercise (1006) objects and/or tasks are presented to the patient in the virtual reality world which encourage the patient to engage in the rehabilitation and/or to remain focused on the exercise and/or to remain entertained. Optionally, and as with other embodiments described herein, measuring (202) and/or displaying (204) are carried out in order to help determine where objects are displayed within the virtual reality environment. In some embodiments of the invention, the at least one sensor 906 is used to track patient movement during exercise (1006) in order to correlate patient movement in the real world to avatar movement in the virtual reality world. In an embodiment of the invention, exercise (1006) includes participation of the upper body of the patient, while the lower body conducts the gait rehabilitation. Upper body participation, for example, involves the patient interacting with objects in the virtual reality environment using at least one finger, hand and/or arm. Exemplary games are described in more detail with respect to FIGS. 8 and 11. In an embodiment of the invention, games are selected (1004) which involve the patient's upper body but also assist with gait training, for example, games which prompt the patient to reach with the upper body which in turn exercises balance, a component of gait. Optionally, patient engagement with the rehabilitation during exercise (1006) is measured, for example by tracking eye gaze, to determine if the game needs to be adjusted to enhance patient engagement, for example by increasing speed, volume, placing more visually stimulating objects in the virtual reality environment, changing the game, etc.

In an embodiment of the invention, assessment (1010) of the patient and/or reporting on the patient's performance and/or progress is conducted. Optionally, assessment and/or reporting is conducted before, during and/or after exercising (1006).

FIGS. 11A-11E are screen shots of representative games, in accordance with exemplary embodiments of the invention. In an embodiment of the invention, FIG. 11A shows a game where the user progresses forward along a path and where different objects (optionally floating) are presented to the user for user selection/touching. FIG. 11B shows a game which combines cognitive abilities with movement abilities, in this embodiment of the invention, the user must move the pieces around in the virtual world to complete the jigsaw puzzle. Another combination cognitive/movement game is shown in FIG. 11C, where the user must match identical cards from memory in order to clear them from the screen. FIG. 11D shows a game where the user must draw a bow and/or aim to shoot balloons/targets floating in the distance. FIG 11E shows a game where during gait training, the user is presented objects for interaction in order to stimulate/motivate the patient during monotonous exercise. In this embodiment, the objects, once selected, fly off the screen in a visually and/or audibly stimulating animated sequence, shown by the residual comet trail of sparkles.

The terms "comprises", "comprising", "includes", "including", "having" and their conjugates mean "including but not limited to".

The term "consisting of" means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims.

All publications, patents and patent applications mentioned in this specification are herein incorporated in their entirety by reference into the specification, to the same extent as if each individual publication, patent or patent application was specifically and individually indicated to be incorporated herein by reference. In addition, citation or identification of any reference in this application shall not be construed as an admission that such reference is available as prior art to the present invention. To the extent that section headings are used, they should not be construed as necessarily limiting.

## Claims

1. A virtual reality based rehabilitation system, comprising:
at least one sensor for detecting and measuring the motion of at least one body portion of a user;
at least one software programmed controller configured to correlate the detected and measured user movement to an avatar of the user in a virtual environment and configured to adapt the virtual reality environment based on the measured motion of the user; and,
at least one output device configured to display the summation of tracked user movement graphically in the virtual environment such that user movement with respect to the user is represented in the virtual environment as avatar movement with respect to the avatar, in the same relative relationship.

2. A system according to claim 1, further comprising a database for storage of at least one of measured motion of at least one user, a virtual reality environment, a billing code, an exercise, a game, patient information, a report, and software.

3. A system according to claim 1 or claim 2, further comprising at least one user interactive device.

4. A system according to claim 3, wherein at least one user interactive device is at least one of an elbow support and a ball.

5. A system according to claim 3, wherein at least one user interactive device is a glove.

6. A system according to any one of claims 1-3, further comprising a connection to at least one external communications network.

7. A system according to any one of claims 1-6, wherein the controller processes measured user range of motion to display objects in a virtual reality environment to the user within the range of motion, near the limit of range of motion or outside the range of motion.

8. A system according to any one of claims 7, wherein the controller presents a game which challenges the user's cognitive and movement abilities in combination.

9. A system according to any one of claims 1-8, wherein the controller assesses user progress based on saved sensor data of the measured motion of the user.

10. A system according to claim 6, wherein at least one of the at least one controller and database is remotely located from the user.

11. A system according to any one of claims 1-10, wherein the same type of coordinate system is used for the user and the avatar.

12. A system according to any one of claims 1-12, wherein the graphically displayed summation of tracked user movement is displayed relative to a reference point.

13. A system according to claim 12, wherein the reference point is at or near the center of the graphically displayed summation of tracked user movement.

14. A system according to claim 12, wherein the reference point is determined in real time.

15. A system according to any one of claims 1-14, wherein the graphic display of the summation of tracked user movement is a bubble where the bubble surface represents the limit of user movement.
